(19) Europäisches Patentamt
European Patent Office
Office européen des brevets

(11) EP 3 397 630 B1

(12) # EUROPEAN PATENT SPECIFICATION

(45) Date of publication and mention of the grant of the patent:
**26.05.2021 Bulletin 2021/21**

(21) Application number: **16822478.0**

(22) Date of filing: **29.12.2016**

(51) Int Cl.:
***C07D 401/12*** *(2006.01)*

(86) International application number:
**PCT/EP2016/082841**

(87) International publication number:
**WO 2017/114898 (06.07.2017 Gazette 2017/27)**

(54) **PROCESS FOR THE PREPARATION OF PANTOPRAZOLE SODIUM SESQUIHYDRATE**
VERFAHREN ZUR HERSTELLUNG VON PANTOPRAZOLE NATRIUM SESQUIHYDRAT
PROCEDE DE PREPARATION DE PANTOPRAZOLE SODIUM SESQUIHDRATE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.12.2015 EP 15203141**
**13.01.2016 SI 201600016 P**

(43) Date of publication of application:
**07.11.2018 Bulletin 2018/45**

(73) Proprietor: **KRKA, tovarna zdravil, d.d., Novo mesto**
**8000 Novo mesto (SI)**

(72) Inventors:
- **KLJAJIC, Alen**
**3000 Celje (SI)**
- **KUFNER, Monika**
**8330 Metlika (SI)**
- **BITENC, Marko**
**1295 Ivancna Gorica (SI)**
- **ZUPET, Rok**
**1000 Ljubljana (SI)**

(74) Representative: **Hoefer & Partner Patentanwälte mbB**
**Pilgersheimer Straße 20**
**81543 München (DE)**

(56) References cited:
**WO-A2-2009/075516 CN-A- 103 467 451**
**US-A1- 2004 186 139 US-B1- 6 410 569**

- **CHEN JIANG ET AL: "Formation Mechanism and Phase Transformation Behaviors of Pantoprazole Sodium Heterosolvate", ORGANIC PROCESS RESEARCH AND DEVELOPMENT, vol. 19, no. 11, 20 November 2015 (2015-11-20), pages 1752-1759, XP055247945, US ISSN: 1083-6160, DOI: 10.1021/acs.oprd.5b00243**
- **JULIE ADAMSON ET AL: "Development of Suitable Plant-Scale Drying Conditions That Prevent API Agglomeration and Dehydration", ORGANIC PROCESS RESEARCH AND DEVELOPMENT, vol. 20, no. 1, 30 November 2015 (2015-11-30), pages 51-58, XP055247822, US ISSN: 1083-6160, DOI: 10.1021/acs.oprd.5b00327**
- **"Glass-lined Filter/Dryers", , 25 September 2008 (2008-09-25), XP055247815, Retrieved from the Internet: URL:http://www.rosenmund.com/FilterTrockner_emailliert_en7301.pdf?cmpref=25462&lang=en&module=media&action=Display [retrieved on 2016-02-05]**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 397 630 B1

## Description

FIELD OF THE INVENTION

The present application relates to a process for the preparation of pantoprazole sodium sesquihydrate.

BACKGROUND OF THE INVENTION

$H^+/K^+$ - ATPase inhibitors, in particular substituted 2-pyridinylmethylsulfinyl-1H-benzimidazoles, are compounds of high importance in the therapy of disorders originating from increased gastric acid secretion. In particular, pantoprazole and pharmaceutically acceptable salts thereof have been beneficially used as gastric acid secretion inhibitors. Pantoprazole is also known under the systematic chemical name 5-(difluoromethoxy)-2-[[(3,4-dimethoxy-2-pyridinyl)methyl]sulfinyl]-1*H*-benzimidazole and its molecular structure is represented by formula (1):

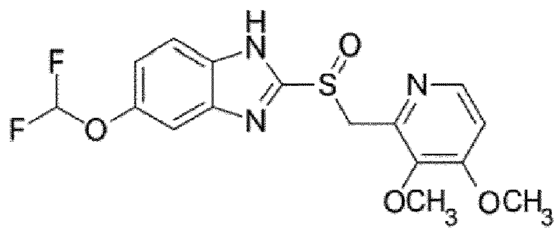

(1)

Various crystalline forms of pantoprazole sodium are known in the art. Examples of methods for the purification of pantoprazole and the formation of pantoprazole salts, such as pantoprazole sodium salts, are in particular disclosed in J. Med. Chemistry 1992, 35, 1049-1057, Anal. Profiles of Drug Substances - Pantoprazole Sodium, Vol. 29, 213-259, Chin. Pharm, August 1999, Vol. 34, No. 8, 564-565, WO2004/080961, IPCOM000016610D of ip.com, Inc., WO 2004/111029, US2004/0186139, WO 2004/ 056804, WO 2004/063188, EP 1 300 406, WO 2007/017244, WO 2006/040778, WO 2007/068925, WO 2007/026188, WO 03/097606, WO 2004/100949 A2 and EP 2 030 973 A1.

In particular, CN 103 467 451 A discloses a preparation method for S-pantoprazole sodium, and a method for preparing amorphous S-pantoprazole sodium.

US 2004/186139 A1 provides a process for making crystalline Form-I of Pantoprazole sodium sesquihydrate.

WO 2009/075516 A2 describes a process for preparation of pantoprazole sodium sesquihydrate from pantoprazole as a starting material.

Chen Jiang et al. describe phase transformation behaviors of pantoprazole sodium heterosolvate in the publication entitled "Formation Mechanism and Phase Transformation Behaviors of Pantoprazole Sodium Heterosolvate", ORGANIC PROCESS RESEARCH AND DEVELOPMENT, vol. 19, 2015, pages 1752-1759.

US 6 410 569 B1 relates to the dihydrate of the magnesium salt of pantoprazole.

Julie Adamson et al. describe drying conditions for alisertib sodium in the publication entitled: "Development of Suitable Plant-Scale Drying Conditions That Prevent API Agglomeration and Dehydration", ORGANIC PROCESS RESEARCH AND DEVELOPMENT, vol. 20, 2016, pages 51 -58.

A publication entitled "Glass-lined Filter/Dryers" of Rosenmund VTA AG, Switzerland, has been described as having been retrieved on 2016-02-05 from the Internet (URL:http://www.rosenmund.com/FilterTrockner_emailliert_en7301.pdf? cmpref=25462&lang=en&module=media&action=Display).

Furthermore, various pantoprazole sodium hydrates, as well as numerous pantoprazole sodium solvates with organic solvents are known in the art. Pantoprazole sodium hydrates are in particular discussed in detail in the International Journal of Pharmaceutics 291 (2005) 59-68. EP 0 533 790 B1 discloses pantoprazole sodium monohydrate. Furthermore, WO 2004/056804 A2 describes crystalline pantoprazole sodium Forms II, IV, V, VI, VIII, IX, X, XI, XII, XIII, XIV, XV, XVI, XVII, XVIII, XIX and XX; pantoprazole sodium solvates containing water, acetone, butanol, methylethylketone, dimethylcarbonate, propanol and 2-methylpropanol, as well as amorphous pantoprazole sodium. Form-I of pantoprazole sodium sesquihydrate and a process for its preparation is disclosed in US 2004/0186139 A1.

DESCRIPTION OF THE INVENTION

The present inventors aimed at providing a process which provides in a simple and reliable manner a pantoprazole sodium hydrate, both on small scale and on industrial scale.

This problem has been solved by the present inventors by the provision of a process for the preparation of pantoprazole sodium sesquihydrate, said process comprising:

a) heating pantoprazole sodium to a temperature in the range from 40°C to 60°C, the pantoprazole sodium being at least partially present in solid form;
b) adding water to the pantoprazole sodium of step a) under agitation to obtain a mixture comprising solid pantoprazole sodium and water;
c) drying the mixture comprising solid pantoprazole sodium and water under agitation; and
d) recovering pantoprazole sodium sesquihydrate.

Advantageously and surprisingly, the preparation process of the present invention allows recovering pantoprazole sodium sesquihydrate. Pantoprazole sodium sesquihydrate is a highly beneficial active agent

which can be used for the preparation of medicaments. Medicaments containing pantoprazole sodium sesquihydrate may be used in particular for treating gastric ulcer, for treating duodenal ulcer, for treating infections with Helicobacter pylori, and for treating Zollinger-Ellison-syndrome, as well as for inhibiting gastric acid secretion.

Furthermore, it is advantageous that the process of the present invention may be used both on small scale, and on an industrial scale. Moreover, the process of the present invention provides a work and time efficient preparation procedure. In particular, the process of the present invention allows that the process may be carried out in situ or as a one-pot-preparation procedure. This minimizes the loss of material resulting from transferring product from one vessel into another vessel.

Furthermore, the process of the present invention may be carried out starting from mixtures comprising pantoprazole sodium and water and/or other solvent(s). This is highly advantageous as it allows to use wet pantoprazole sodium directly as starting material without having to carry out a work and energy consuming drying procedure.

The process of the present invention comprises heating pantoprazole sodium to a temperature of 40°C to 60°C. This heating of pantoprazole sodium in step a) can be carried out for a time period of at least 1 minute, preferably for a time period of at least about 3 minutes to about 24 hours, more preferably for a time period of about 0.4 hours to about 3 hours, especially for a time period of about 0.5 hours to about 2 hours.

The pantoprazole sodium subjected to heating in step a) can be or can comprise pantoprazole sodium selected from the group consisting of pantoprazole sodium solvates, especially pantoprazole sodium hydrates and pantoprazole sodium solvates containing at least one organic solvent and optionally water, pantoprazole sodium in water-free form, and mixtures thereof; especially pantoprazole sodium can be selected from the group consisting of pantoprazole sodium hydrates (such as in particular pantoprazole sodium monohydrate), pantoprazole sodium solvates containing at least one organic solvent and optionally water, and mixtures thereof.

In particular, the pantoprazole sodium solvate containing at least one organic solvent and optionally water can contain at least one organic solvent selected from the group consisting of ketones (such as e.g. ketones of formula $R^1$-C(O)-$R^2$, with $R^1$ and $R^2$ being independently selected from C1-C4 alkyl, in particular e.g. acetone, ethyl-methyl-ketone, methyl-isobutyl-ketone, diethyl-ketone), C1-C4 alcohols (such as e.g. butanol, propanol and 2-methylpropanol), aliphatic esters (such as e.g. esters of formula $R^3$-CCO)-O-$R^4$, with $R^3$ and $R^4$ being independently selected from C1-C4 alkyl, in particular e.g. ethyl acetate, propyl acetate, isopropyl acetate, butyl acetate, (e.g. n- or sec- or tert-butyl acetate), dimethylcarbonate, and mixtures thereof.

Especially, the pantoprazole sodium subjected to heating in step a) can be or can comprise at least one pantoprazole sodium selected from the group consisting of Form A, Form B, monohydrate, sesquihydrate as identified in the International Journal of Pharmaceutics 291 (2005) 59-68 ("Physical characterization of pantoprazole sodium hydrates", authors: V. Zupančič, N. Ograjšek, B. Kotar-Jordan, F. Vrečer), pantoprazole sodium Forms II, IV, V, VI, VIII, IX, X, XI, XII, XIII, XIV, XV, XVI, XVII, XVIII, XIX and XX as identified in WO 2004/056804 A2, Form-I of pantoprazole sodium sesquihydrate as identified in US 2004/0186139 A1, especially having a X-ray powder diffraction pattern as shown in Figure 1 thereof, pantoprazole sodium sesquihydrate having a X-ray powder diffraction pattern as shown in Figure 1 of EP 2 030 973 A1, pantoprazole sodium monohydrate, especially pantoprazole sodium monohydrate "which on account of its water content can be described roughly as monohydrate" as identified in EP 0 533 790 B1, Forms N, A1, A2, A3, A4, B1, B2, B3, C1, C2, D1, E1, as identified in WO 2004/100949 A2, and mixtures thereof. Examples of methods for the purification of pantoprazole and the formation of pantoprazole salts, such as pantoprazole sodium salts, are in particular disclosed in J. Med. Chemistry 1992, 35, 1049-1057, Anal. Profiles of Drug Substances - Pantoprazole Sodium, Vol. 29, 213-259, Chin. Pharm, August 1999, Vol. 34, No. 8, 564-565, WO2004/080961, IPCOM000016610D of ip.com, Inc., WO 2004/111029, US2004/0186139, WO 2004/ 056804, WO 2004/063188, EP 1 300 406, WO 2007/017244, WO 2006/040778, WO 2007/068925, WO 2007/026188, WO 03/097606, and EP 2 030 973 A1. Pantoprazole sodium acetone water solvates can be in particular prepared as exemplified in WO 2004/100949 A2. WO 2004/100949 A2, EP 2 030 973 A1, EP 0 533 790 B1, WO 2004/056804 A2, and Journal of Pharmaceutics 291 (2005) 59-68.

Particularly advantageous procedural results were obtained when subjecting pantoprazole sodium hydrates, and/or pantoprazole sodium ketone solvates (especially acetone solvates) and/or pantoprazole sodium ketone water solvates (especially acetone water solvates) to heating in step a).

Preferably, the pantoprazole sodium subjected to heating in step a) can be or can comprise pantoprazole sodium selected from the group consisting of pantoprazole sodium hydrates (such as in particular pantoprazole sodium monohydrate), pantoprazole sodium solvates containing at least one ketone and optionally water (such as in particular pantoprazole sodium acetone solvates and pantoprazole sodium acetone water solvates), and mixtures thereof; preferably, said at least one ketone can be at least one ketone selected from the group consisting of ketones of formula $R^1$-C(O)-$R^2$, with $R^1$ and $R^2$ being independently selected from methyl, ethyl, propyl, butyl, in particular e.g. acetone, ethyl-methyl-ketone, methyl-isobutyl-ketone, diethyl-ketone, and mixtures thereof; preferably said at least one ketone can be acetone.

Especially, the pantoprazole sodium subjected to heating in step a) can be or can comprise pantoprazole sodium selected from the group consisting of pantoprazole sodium hydrates (such as in particular pantoprazole sodium monohydrate), pantoprazole sodium acetone solvates, pantoprazole sodium acetone water solvates, and mixtures thereof.

Pantoprazole sodium monohydrate can be in particular prepared as exemplified in the Example section hereinafter, or can be prepared as described in EP 0 533 790 B1, especially according to one of the procedures explained in the Example part thereof, or can be a monohydrate according to International Journal of Pharmaceutics 291 (2005) 59-68 ("Physical characterization of pantoprazole sodium hydrates", authors: V. Zupančič, N. Ograjšek, B. Kotar-Jordan, F. Vrečer).

Pantoprazole sodium acetone solvates and/or pantoprazole sodium acetone water solvates can be in particular prepared as exemplified in the Example section hereinafter, or can be prepared as described in in WO 2004/100949 A2. Pantoprazole sodium acetone water solvates can for example consist of or can comprise at least one of acetone solvate sodium aqua complexes Form A1, Form A2, Form A3 and Form A4 (as disclosed and prepared in WO 2004/100949 A2).

The term "pantoprazole sodium acetone solvate" may refer to any pantoprazole sodium solvate containing acetone, as well as to mixtures of these solvates. The term "solvate containing solvent X" can have in particular the meaning that the solvate contains solvent X as solvate forming solvent.

The term "pantoprazole sodium acetone water solvate" may refer to any pantoprazole sodium solvate containing water and acetone and to mixtures of such solvates, as well as to mixtures of one or more pantoprazole sodium hydrate(s) and one or more pantoprazole sodium solvate(s) containing acetone and optionally water.

Furthermore, the term "pantoprazole sodium acetone water solvate" may refer to pantoprazole sodium solvate(s) obtained (e.g. precipitated, especially crystallized) from a mixture comprising or consisting of pantoprazole sodium, water and acetone.

Preferably, the term "pantoprazole sodium acetone water solvate" may refer to any pantoprazole sodium solvate containing water and acetone, as well as to mixtures of pantoprazole sodium solvates containing water and acetone.

In particular, the pantoprazole sodium acetone water solvate can be selected from the group consisting of acetone solvate sodium aqua complexes Form A1, Form A2, Form A3 and Form A4 (as disclosed and prepared in WO 2004/100949 A2), and mixtures thereof, and from pantoprazole sodium acetone water solvate mixtures comprising at least one of acetone solvate sodium aqua complexes Form A1, Form A2, Form A3 and Form A4.

Especially, the pantoprazole sodium subjected to heating in step a) can be or can comprise one or more pantoprazole sodium solvates, in particular can be or comprise at least one of one or more pantoprazole sodium hydrates, one or more pantoprazole sodium solvates containing at least one organic solvent and optionally water, and mixtures thereof.

In one preferred embodiment, the pantoprazole sodium subjected to heating in step a) can be or can comprise a pantoprazole sodium monohydrate. In another preferred embodiment, the pantoprazole sodium subjected to heating in step a) can be or can comprise one or more pantoprazole sodium solvate(s) containing one or both of water and acetone, especially containing acetone and optionally water, further especially containing acetone and water.

In particular, the pantoprazole sodium hydrate subjected to heating in step a) can be a pantoprazole sodium hydrate selected from the group consisting of pantoprazole sodium monohydrate, pantoprazole sodium hemihydrate, pantoprazole sodium sesquihydrate, pantoprazole sodium dihydrate, or a mixture comprising at least two of these pantoprazole sodium hydrates.

The terms "pantoprazole sodium", "pantoprazole sodium solvate" and "pantoprazole sodium hydrate", respectively, encompass crystalline form(s) and amorphous form(s). A pantoprazole sodium solvate wherein the solvent forming the solvate is water can be also referred to herein as pantoprazole sodium hydrate. A pantoprazole sodium solvate wherein the solvent forming the solvate is acetone can be also referred to herein as pantoprazole sodium acetone solvate.

The temperature of the pantoprazole sodium can be determined by a temperature measuring device (such as e.g. a liquid-in-glass thermometer (for example Hg-in-glass thermometer, alcohol thermometer), thermocouple, ...) which may be chosen by the skilled person on the basis of the general knowledge in the art. In particular, the temperature of the pantoprazole sodium can be determined on the surface of the pantoprazole sodium or the mixture comprising pantoprazole sodium.

The pantoprazole sodium subjected to heating is pantoprazole sodium which is at least partially present in solid form. Prior to step a) a step of obtaining pantoprazole sodium which is at least partially present in solid form can be carried out. This step is also referred to herein as step 1). In this step prior to step a), for example solvent may be removed (e.g. by evaporation) from a solution comprising pantoprazole sodium and a solvent in order to obtain a pantoprazole sodium which is at least partially present in solid form, and/or crystallisation may be induced. Inducing crystallisation can be or comprise one or both of the following procedural steps: adding solvent(s) inducing the precipitation of pantoprazole sodium (especially pantoprazole sodium monohydrate or pantoprazole sodium acetone water solvate(s)), and adding seed crystals (especially pantoprazole sodium monohydrate seed crystals or pantoprazole sodium acetone water solvate seed crystals). The solution comprising pantoprazole sodium and a solvent can be in particular a solution comprising water, or a solution comprising at least one organic solvent, or a solution comprising a mixture of water and at least one organic solvent; preferably said at least one organic solvent can be selected from the group consisting of ketones (such as e.g. ketones of formula $R^1$-C(O)-$R^2$, with $R^1$ and $R^2$ being independently selected from C1-C4 alkyl, in particular e.g. acetone, ethyl-methyl-ketone, methyl-isobutyl-ketone, diethyl-ketone), C1-C4 alcohols (such as e.g. butanol, propanol and 2-methylpropanol), aliphatic esters (such as e.g. esters of formula $R^3$-CCO)-O-$R^4$, with $R^3$ and $R^4$ being independently selected from C1-C4 alkyl, in particular e.g. ethyl acetate, propyl acetate, isopropyl acetate, butyl acetate, (e.g. n- or sec- or tert-butyl acetate), dimethylcarbonate, and mixtures thereof.

Especially, step 1) of obtaining pantoprazole sodium which is at least partially present in solid form can comprise preparing pantoprazole sodium solvate(s) being at least partially present in solid form, especially can comprise preparing pantoprazole sodium hydrate(s) being at least partially present in solid form, and/or preparing pantoprazole sodium solvates containing at least one organic solvent and optionally water being at least partially present in solid form, in particular can comprise preparing pantoprazole sodium monohydrate being at least partially present in solid form and/or preparing pantoprazole sodium acetone-water solvate(s) being at least partially present in solid form.

Preparing pantoprazole sodium being at least partially present in solid form can comprise:

reacting pantoprazole with an aqueous mixture comprising NaOH, said aqueous mixture further preferably comprising at least one organic solvent (especially acetone) to obtain a reaction mixture;
optionally filtering the reaction mixture to obtain a reaction solution;
inducing precipitation of pantoprazole sodium (especially of pantoprazole sodium acetone-water solvates and/or pantoprazole sodium acetone solvates and/or pantoprazole sodium hydrates, such as e.g. pantoprazole sodium monohydrate) in the reaction mixture, which has been optionally filtered;
recovering pantoprazole sodium being at least partially present in solid form, preferably by subjecting the reaction mixture to centrifugation or pressure filter treatment. The at least one organic solvent can be selected from the group consisting of ketones (such as e.g. ketones of formula $R^1$-CCO)-$R^2$, with $R^1$ and $R^2$ being independently selected from C1-C4 alkyl, in particular e.g. acetone, ethyl-methyl-ketone, methyl-isobutyl-ketone, diethyl-ketone), C1-C4 alcohols (such as e.g. butanol, propanol and 2-methylpropanol), aliphatic esters (such as e.g. esters of formula $R^3$-CCO)-O-$R^4$, with $R^3$ and $R^4$ being independently selected from C1-C4 alkyl, in particular e.g. ethyl acetate, propyl acetate, isopropyl acetate, butyl acetate, (e.g. n- or sec- or tert-butyl acetate), dimethylcarbonate, and mixtures thereof. The pantoprazole sodium recovered can be present in a mixture comprising at least one or both of water and at least one organic solvent (especially in a mixture comprising at least one or both of water and acetone). This mixture may be directly used in the process of the present invention. For example mixtures containing at least 10 wt.-%, preferably at least 50 wt.-%, more preferably at least 60 wt.-%, especially at least 80 wt.-%, in particular at least 90 wt.-% of pantoprazole sodium, each based on the weight of the mixture, can be used in step a) of the process of the present invention.

Preparing pantoprazole sodium acetone-water solvate(s) can comprise:

i) reacting pantoprazole with an aqueous mixture comprising NaOH, said aqueous mixture further comprising acetone, preferably at a temperature in the range of 40°C-60°C, more preferably in the range of 40°C-45°C, to obtain a reaction mixture;
ii) optionally adding active carbon to the reaction mixture;
iii) optionally heating the reaction mixture to a temperature in the range of 50°C to 60°C;
iv) optionally filtering the reaction mixture;
v) inducing precipitation of pantoprazole sodium in the reaction mixture (obtained according to one of steps i) to iv), especially of pantoprazole sodium acetone-water solvates, preferably by
cooling the reaction mixture, in particular cooling to a temperature of 5°C or less, especially to a temperature in the range form 0°C to 5°C, wherein the cooling of the mixture can be preferably carried out at a cooling rate of 0.1 to 0.8°C/minute, and
optionally adding pantoprazole sodium seeding crystals (especially pantoprazole sodium monohydrate seeding crystals and/or pantoprazole sodium acetone-water solvate seeding crystals), preferably said adding being carried out during the cooling step when the mixture comprising pantoprazole sodium has reached a temperature of in the range from 40 to 42°C;
vi) recovering pantoprazole sodium comprising or consisting of pantoprazole sodium acetone-water solvate(s) and being at least partially present in solid form, preferably by subjecting the reaction mixture to centrifugation or pressure filter treatment.

The pantoprazole sodium recovered can be present in a mixture comprising one or both of water and acetone. This mixture may be directly used in the process of the present invention. For example mixtures containing at least 10 wt.-%, preferably at least 50 wt.-%, more preferably at least 60 wt.-%, especially at least 80 wt.-%, in particular at least 90 wt.-% of pantoprazole sodium, each based on the weight of the mixture, can be used in step a) of the process of the present invention.

Preparing pantoprazole sodium monohydrate

can comprise:
carrying out steps i) to iv) as indicated supra,

v) inducing precipitation of pantoprazole sodium monohydrate in the reaction mixture (obtained according to one of steps i) to iv), preferably by cooling the reaction mixture, in particular cooling to a temperature of 5°C or less, especially to a temperature in the range form 0°C to 5°C, wherein the cooling of the mixture can be preferably carried out at a cooling rate of 0.1 to 0.8°C/minute, and
adding pantoprazole sodium monohydrate seeding crystals, preferably said adding being carried out during the cooling step when the mixture comprising pantoprazole sodium has reached a temperature of in the range from 40 to 42°C;
vi) recovering pantoprazole sodium being at least partially present in solid form, preferably by subjecting the reaction mixture to centrifugation or pressure filter treatment, and optionally washing the recovered pantoprazole sodium,
viii) optionally drying the pantoprazole sodium obtained in step vi) to obtain dry pantoprazole sodium monohydrate, preferably the drying being carried out at a temperature in the range of 20°C to 45°C, more preferably the drying comprises a first drying step at a temperature in the range of 20°C to 25°C, and a second drying step, wherein the temperature is increased from a temperature of 25°C to a temperature of 45°C; the drying can be e.g. carried out at a pressure reduced below ambient pressure, for example at a pressure of 80 kPa or less.

Pantoprazole sodium monohydrate seeding crystals can be obtained by one of the processes described herein, especially in the Example section or can be e.g. obtained by one of the processes for preparing pantoprazole sodium monohydrate disclosed in EP 0 533 790 B1 or in further publications. Pantoprazole sodium acetone-water solvate seeding crystals can be obtained by one of the processes described herein, especially in the Example section or can be e.g. seeding crystals of one of acetone solvate sodium aqua complexes Form A1, Form A2, Form A3 and Form A4 (as disclosed and prepared in WO 2004/100949 A2).

In one embodiment, the pantoprazole sodium hydrate subjected to heating in step a) can be present as a solid starting material, in particular as a solid starting material consisting of pantoprazole sodium or comprising at least 90 wt.-%, especially at least 95 wt.-%, in particular at least 98 wt.-% of pantoprazole sodium, based on the total weight of the starting material. This starting material can be preferably present in form of a powder, especially in form of a powder consisting of particles having a particle size of less than 1 mm, preferably less than 500 μm, more preferably less than 200 μm. Particle size can be in particular determined by analytical sieving. Particle size determination can be carried out as described in the US Pharmacopoeia's (USP) publication USP-NF (2004 - Chapter 786 (The United States Pharmacopoeial Convention, Inc., Rockville, MD)).

Furthermore, the pantoprazole sodium to be subjected to heating in step a) can be present in a mixture comprising at least one solvent and pantoprazole sodium, preferably in a mixture comprising at least one solvent and solid pantoprazole sodium, more preferably in a mixture comprising at least one solvent and one or more pantoprazole sodium solvates, especially in a mixture comprising at least one solvent and at least one of one or more pantoprazole sodium hydrates, one or more pantoprazole sodium solvates containing at least one organic solvent and optionally water, and mixtures thereof. In these mixtures to be subjected to heating in step a), the pantoprazole sodium being at least partially present in solid form.
The mixture comprising at least one solvent can be preferably a mixture comprising at least one solvent selected from water and organic solvents, in particular a mixture comprising at least one solvent selected from water, ketones (such as e.g. ketones of formula $R^1$-C(O)-$R^2$, with $R^1$ and $R^2$ being independently selected from C1-C4 alkyl, in particular e.g. acetone, ethyl-methyl-ketone, methyl-isobutyl-ketone, diethyl-ketone), C1-C4 alcohols (such as e.g. butanol, propanol and 2-methylpropanol), dimethylcarbonate, aliphatic esters (such as e.g. esters of formula $R^3$-CCO)-O-$R^4$, with $R^3$ and $R^4$ being independently selected from C1-C4 alkyl, in particular e.g. ethyl acetate, propyl acetate, isopropyl acetate, butyl acetate, (e.g. nor sec- or tert-butyl acetate), and mixtures thereof. Preferably, said at least one solvent is at least one or both of water and acetone, and optionally one or more further solvents.

An organic solvent can be in particular an organic compound which is liquid at room temperature (preferably 20°C) and ambient pressure (preferably 1 atm (101.325 kPa)). An organic compound can be in particular a compound containing at least one carbon atom.

In particular, the pantoprazole sodium to be subjected to heating in step a) can be present in a mixture comprising pantoprazole sodium, at least one organic solvent, and optionally water. The at least one organic solvent can be selected from the group consisting of ketones (such as e.g. ketones of formula $R^1$-C(O)-$R^2$, with $R^1$ and $R^2$ being independently selected from C1-C4 alkyl, in particular e.g. acetone, ethyl-methyl-ketone, methyl-isobutyl-ketone, diethyl-ketone), C1-C4 alcohols (such as e.g. butanol, propanol and 2-methylpropanol), aliphatic esters (such as e.g. esters of formula $R^3$-CCO)-O-$R^4$, with $R^3$ and $R^4$ being independently selected from C1-C4 alkyl, in particular e.g. ethyl acetate, propyl acetate, isopropyl acetate, butyl acetate, (e.g. n- or sec- or tert-butyl acetate), dimethyl carbonate, and mixtures thereof. Most preferred is acetone. Said mixture comprising pantoprazole sodium, at least one organic solvent, and optionally water may contain less than 95 wt.-%, in particular less than 90 wt.-%, preferably less

than 50 wt.-%, more preferably less than 40 wt.-%, optionally at least 0.1 wt.-%, further optionally at least 1 wt.% or at least 3 wt.% of the at least one organic solvent, each based on the weight of the mixture.

In particular, the pantoprazole sodium to be subjected to heating in step a) can be present in a mixture comprising pantoprazole sodium, acetone, and optionally water, said mixture containing less than 95 wt.-% of acetone, in particular less than 90 wt.-% of acetone, preferably less than 50 wt.-% of acetone, more preferably less than 40 wt.-%, especially between less than 40 wt.-% and at least 0.1 wt.-% of acetone, each based on the weight of the mixture.

During the heating of pantoprazole sodium in step a), the pantoprazole sodium can be subjected to agitation. In particular, the pantoprazole sodium can be subjected to an agitation (e.g. a stirring) of at least 0,5 rpm (revolutions per minute), in particular of at least 2 rpm (revolutions per minute), especially of at least 10 rpm (revolutions per minute), for example of at least 2 rpm and up to 20 rpm. Subjecting the pantoprazole sodium to agitation can be carried out by any means known to the skilled person, such as e.g. by a mechanical stirrer, e.g. in a filter dryer comprising a stirrer, or by a mixer, especially a high shear mixer. High shear mixers can be in particular mixers capable of operating at mixing speeds encompassing the range of 0,3 to 10000 rpm (or higher), preferably encompassing the range of 5 rpm to 4000 rpm (or higher).

Subjecting the pantoprazole sodium to agitation prevents an agglomeration of the pantoprazole sodium to agglomerates, and facilitates the heat transfer within the pantoprazole sodium to be subjected to heating in step a).

After having heated pantoprazole sodium to a temperature of at least 30°C, water is added to the pantoprazole sodium heated to a temperature of at least 30°C. The adding of water to the pantoprazole sodium is carried under agitation to obtain a mixture comprising solid pantoprazole sodium and water. The adding of water to the pantoprazole sodium heated to a temperature of at least 30°C under agitation can be carried out during a water addition time period; this water addition time period can be a time period of at least 1 minute, preferably at least 4 minutes, more preferred at least 10 minutes, for example a time period from at least 1 minute to 20 hours, especially from 10 minutes to 50 minutes.

The water added (in step b)) can have a temperature of e.g. at least 10°C, in particular at least 30°C, preferably at least 35°C, more preferably at least 40°C, especially a temperature in the range from 40°C to 60°C

The amount of water added in step b) (during the water addition time period) can be at least 1 wt.-%, preferably at least 5 wt.-%, more preferably at least 10 wt.-%, more preferably at least 15 wt.-%, especially in the range of 1 to 80 wt.-%, especially in the range of 1 to 30 wt.-%, each based on the total weight of pantoprazole sodium.

During step b), the pantoprazole sodium and/or the mixture comprising pantoprazole sodium can be subjected to agitation. In particular, the agitation can be an agitation (e.g. a stirring) of at least 1 rpm (revolutions per minute), in particular of at least 25 rpm (revolutions per minute), especially of at least 30 rpm (revolutions per minute), preferably of at least 30 rpm and e.g. up to 40 rpm. Subjecting to agitation can be carried out by any means known to the skilled person, such as e.g. by a mechanical stirrer, e.g. in a filter dryer comprising a stirrer, or by a mixer, especially a high shear mixer.

Subjecting to agitation prevents an agglomeration of the pantoprazole sodium to agglomerates, facilitates obtaining a mixture of high homogenousity, and facilitates the heat transfer within the mixture. Preferably, the mixture obtained after having completed the water addition in step b) is a homogenous mixture.

The adding of water to the pantoprazole sodium can be carried out continuously or intermittently. The water addition time period extends from the contacting of the pantoprazole sodium with the first amount (e.g. drop) of added water until the contacting of the mixture comprising pantoprazole sodium and water with the last amount of added water, irrespective of whether the water addition is interrupted for one or more time periods or not. The water addition may be e.g. interrupted for 2, 3, 4, 5 or ten or more times during water addition time period. If desired, the amount of water per minute may be varied, in particular increased or decreased, during one or more phases of the water addition time period.

The temperature in step b) and/or in step c) can be adjusted to a temperature of at least 30°C, preferably at least 35°C, more preferably at least 40°C, especially to a temperature in the range from 40°C to 60°C.

In particular, the adding of water according to step b) can be or can comprise spraying the water onto the pantoprazole sodium. By spraying the water onto the pantoprazole sodium, the water is distributed in small amounts. This allows that the water can be advantageously and easily mixed with the pantoprazole sodium.

Step b) can be carried out completely or partially (e.g. during at least 50% of the water addition time period) under an atmosphere having a relative humidity of more than 80%, more preferably of at least 83%, even more preferably of at least 86%, especially of at least 90%, in particular of at least 90%.

During step b) (or during the water addition time period) an amount of water can be added to the pantoprazole sodium which can be e.g. in the range of $0.1\,\frac{L}{kg \cdot h}$ to $0.8\,\frac{L}{kg \cdot h}$, in particular e.g. in the range of $0.2\,\frac{L}{kg \cdot h}$ to $0.5\,\frac{L}{kg \cdot h}$ (Liter of water per hour and per kg of pantoprazole sodium).

Optionally, step b) of adding water may furthermore comprise adding seeding crystals of pantoprazole

sodium sesquihydrate. Pantoprazole sodium sesquihydrate seeding crystals can be obtained by one of the processes described herein, especially in the Example section, or can be e.g. obtained by a process disclosed in US 2004/0186139 A1 or in another publication.

In particular, the temperature of the mixture comprising pantoprazole sodium and water which is formed upon addition of water to the heated pantoprazole can be adjusted during the water addition time period to a temperature of at least 30°C, preferably of at least 35°C, more preferably of at least 40°C, especially to a temperature in the range from 40°C to 60°C. The mixture comprising pantoprazole sodium and water obtained at the end of the water addition period can have a temperature of at least 30°C, preferably at least 35°C, more preferably at least 40°C, especially in the range from 40°C to 60°C.

The mixture comprising solid pantoprazole sodium and water resulting from step b) of adding water to the pantoprazole sodium can be subjected to a drying under agitation.

In particular, the temperature of the mixture comprising pantoprazole sodium and water can be during drying within a temperature range of at least 30°C, preferably at least 35°C, more preferably at least 40°C, especially in the range from 40°C to 60°C.

During drying the pantoprazole can be subjected to an agitation (e.g. a stirring). The agitation can be an agitation (e.g. a stirring) of for example at least 0,5 rpm (revolutions per minute), in particular of at least 5 rpm (revolutions per minute), further in particular of at least 10 rpm (revolutions per minute).

Preferably, the drying of the mixture comprising solid pantoprazole sodium and water is or comprises contacting the mixture with a gas flow, which gas flow removes water and optionally residual solvent(s) from the mixture. By flowing gas over the mixture, water and optionally residual solvent molecules can be incorporated in gaseous form into the gas flow passing over the mixture, and thereby removed from the mixture. The drying can be carried out e.g. by means of a gas flow comprising or consisting of a gas selected from the group consisting of ambient air, nitrogen, noble gases, and mixtures thereof. The removal of the water and optionally residual solvent(s) from the mixture can be facilitated by adjusting the temperature of the mixture to a temperature of at least 30°C, preferably at least 35°C, more preferably at least 40°C, especially to a temperature in the range from 40°C to 60°C. Composition of the gas and water content of the gas can be determined by the skilled person on the basis of the general knowledge in the art. Procedures for reducing the water content of a gas flow, if necessary, are known to the skilled person.

Furthermore, the drying of the mixture comprising solid pantoprazole sodium and water can be carried out at a pressure reduced below ambient pressure, preferably at a pressure of 80 kPa or less, more preferably 75 kPa or less, even more preferably at a pressure 60 kPa or less, especially at a pressure in the range $10^{-4}$ kPa to 55 kPa, in particular at a pressure in the range 30 kPa to 52 kPa. Optionally, the temperature of the mixture comprising pantoprazole sodium and water subjected to drying (in step c)) under reduced pressure can be during drying within a temperature range of at least 30°C, preferably at least 35°C, more preferably at least 40°C, especially to a temperature in the range from 40°C to 60°C.

In particular, the drying may be or may comprise a drying under reduced pressure in combination with contacting the mixture with a gas flow for removing water and optionally residual solvent(s) from the mixture, preferably the temperature of the mixture comprising pantoprazole sodium and water being during drying within a temperature range of at least 30°C, preferably at least 35°C, more preferably at least 40°C, especially in the temperature range from 40°C to 60°C.

The step of drying of the mixture comprising solid pantoprazole sodium and water can be carried out e.g. during a time period of at least 2 hours, optionally during a time period from 6 hours to 10 days.

The drying of the mixture comprising solid pantoprazole sodium and water can be carried out until the mixture comprising solid pantoprazole sodium and water has a water content of below 7,5 wt.-%, preferably below 7 wt.%, each based on the weight of pantoprazole sodium present in the mixture, determined by Karl-Fischer method. Karl-Fischer method can be carried out according to Ph. Eur. 2.5.12 (e.g. according to European Pharmacopoeia 7.0, chapter 2.5.12) or USP 921 (US Pharmacopoeia). The water content can be calculated on the basis of pantoprazole sodium in non-solvated form. The drying can be stopped when all water in free form, preferably all water in free form and all solvents other than water, has/have been removed from the pantoprazole sodium sesquihydrate subjected to drying. As used herein, "water in free form" is distinct from water bound in solvate (hydrate) form and does not encompass water bound in solvate (hydrate) form.

A particular advantage of the process of the present invention is that the process may be carried out in situ, i.e. in a "one-pot-preparation procedure". In particular, all steps a) to c) of the present invention can be carried out in the same container, e.g. in a filter dryer, especially in a filter dryer comprising a stirrer, or in a high shear mixer. Especially, all steps a) to c) of the present invention can be carried out in the same container, which container is provided with a stirrer. This stirrer can be adapted to subject the pantoprazole sodium or the mixture present in the container to agitation.

The pantoprazole sodium sesquihydrate recovered in the process of the present invention (step d)) can be in particular pantoprazole sodium sesquihydrate as identified in US 2004/0186139 A1 as Form-I, especially pantoprazole sodium sesquihydrate having a powder X-ray diffraction pattern substantially as depicted in Figure 1 of US 2004/0186139 A1. The X-ray powder diffractogram of the crystalline Form-I of pantoprazole sodium sesquihydrate was measured in US 2004/0186139 A1

with Cu K alpha-1 Radiation source, especially on a Bruker Axs, D8 Advance Powder X-ray Diffractometer with Cu K alpha-1 Radiation source. The pantoprazole sodium sesquihydrate recovered in the process of the present invention (step d)) can be in particular pantoprazole sodium sesquihydrate characterized by a powder X-ray diffraction pattern having peaks at 5.238, 7.294, 10.523, 11.428, 12.345, 13.024, 13.30, 14.459, 15.207, 15.844, 16.15, 16.653, 17.527, 17.884, 18.404, 19.97, 20.486, 21.214, 22.148, 22.812, 23.26, 24.002, 24.497, 24.957, 25.32, 26.278, 27.037, 27.798, 28.737, 28.383, 30.18, 30.861, 32.652, 33.647, 34.671 and 37.572 degrees two-theta or having at least eight peaks thereof; the X-ray powder diffractogram can be determined with Cu K alpha-1 Radiation source, especially the X-ray powder diffractogram can be measured on a Bruker Axs, D8 Advance Powder X-ray Diffractometer with Cu K alpha-1 Radiation source. US 2004/0186139 A1.

The process of the present invention is a process which allows a work and time efficient preparation of pantoprazole sodium sesquihydrate. This is unexpected and surprising, as not merely one, but several hydrates of pantoprazole sodium exist.

In particular, in a process for the preparation of pantoprazole sodium sesquihydrate of the present invention, the pantoprazole sodium to be subjected to heating in step a) can comprise or can consist of pantoprazole sodium present in a form different from pantoprazole sodium sesquihydrate. Carrying out steps a) to c) can convert pantoprazole sodium present in a form (especially crystalline form) different from pantoprazole sodium sesquihydrate into pantoprazole sodium sesquihydrate. Said conversion of pantoprazole sodium present in a form different from pantoprazole sodium sesquihydrate into pantoprazole sodium sesquihydrate can be a partial conversion or can be an essentially complete or a complete conversion into pantoprazole sodium sesquihydrate.

According to one embodiment, carrying out steps a) to c) converts pantoprazole sodium present in a form different from pantoprazole sodium sesquihydrate into pantoprazole sodium sesquihydrate, wherein at least 10 mole %, in particular at least 30 mole %, further in particular at least 50 mole %, further in particular at least 80 mole %, further in particular at least 95 mole %, further in particular at least 99 mole % of the pantoprazole sodium to be subjected to heating in step a) which is present in a form different from pantoprazole sodium sesquihydrate can be converted into pantoprazole sodium sesquihydrate, based on the total number of moles of pantoprazole sodium to be subjected to heating in step a) which is present in a form different from pantoprazole sodium sesquihydrate.

According to a preferred embodiment, carrying out steps a) to c) converts all or essentially all of the pantoprazole sodium to be subjected to heating in step a) which is present in a form different from pantoprazole sodium sesquihydrate into pantoprazole sodium sesquihydrate.

In a process for the preparation of pantoprazole sodium sesquihydrate of the present invention, the pantoprazole sodium to be subjected to heating in step a) can be pantoprazole sodium free of pantoprazole sodium sesquihydrate.

In a process for the preparation of pantoprazole sodium sesquihydrate of the present invention, the pantoprazole sodium to be subjected to heating in step a) can be pantoprazole sodium comprising less than 98 mole %, in particular less than 90 mole %, especially less than 60 mole %, further especially less than 20 mole %, still further especially less than 10 mole % of pantoprazole sodium sesquihydrate, based on the total number of moles of pantoprazole sodium.

In a process for the preparation of pantoprazole sodium sesquihydrate of the present invention, pantoprazole sodium to be subjected to heating in step a) can be a mixture comprising one or more pantoprazole sodium solvates, the amount of pantoprazole sodium sesquihydrate in said mixture being less than 98 mole %, in particular less than 90 mole %, especially less than 60 mole %, further especially less than 20 mole %, still further especially less than 10 mole %, based on the total number of moles of pantoprazole sodium in said mixture.

In the following, the present invention will be illustrated by several non-limiting examples.

## Examples

Example 1: Preparation of pantoprazol sodium sesquihydrate

Pantoprazol sodium salt monohydrate powder (4 kg, obtained in Example 10), was charged into preheated filter dryer (Rosenmund, 0,1 $m^2$, jacket temperature set to 60°C) equipped with pneumatic-water spray nozzle and mechanical stirrer. Wet material was preheated in filter dryer with jacket temperature 60°C and constant stirring (10-20 rpm) for 0,5 hours before water was added. Water (0.95 L) was sprayed on powder with constant flow in 0.5 hours at constant stirring speed (30-40 rpm). Afterwards the wetted powder is dried under reduced pressure (~500 mbar), jacket temperature 60°C and constant air flow until water content in the powder below 7 % is reached. The dried product (3.8 kg) was obtained in the form of powder after 20 hours of drying (LOD = 0.2%, KF = 6.0%, sesquihydrate polymorphic form).

Example 2: Preparation of pantoprazol sodium sesquihydrate

Pantoprazol sodium salt monohydrate powder (8 kg, obtained in Example 9), was charged into preheated filter dryer (Rosenmund, 0,1 $m^2$, 60°C) equipped with pneumatic-water spray nozzle and mechanical stirrer. Wet material was pre-heated in filter dryer with jacket temperature 60°C and constant stirring (10-20 rpm) for

1,25 hours before water spraying. Water (1,5 L) was sprayed on powder with constant flow in 0.75 hours at constant stirring speed (30-40 rpm). Afterwards the wetted powder is dried under reduced pressure (~500 mbar), jacket temperature 60°C and constant air flow until water content in the powder below 7 % is reached. The dried product (7.8 kg) was obtained in the form of powder after 12 hours of drying (LOD = 0.5%, KF = 6.5%, sesquihydrate polymorphic form).

Example 3: Preparation of pantoprazol sodium sesquihydrate

Wet pantoprazol sodium salt (8.9 kg, LOD 26.6%, KF 7.4%, obtained in Example 7), was charged into preheated filter dryer (Rosenmund, 0,1 $m^2$, 60°C) equipped with pneumatic-water spray nozzle, mechanical stirrer and open valves for venting acetone vapors. Wet material was pre-heated in filter dryer with jacket temperature 60°C and minimal stirring (cca 2-3 rpm) for 2 hours before water spraying. Water (1 L) was sprayed on powder with constant flow in 0.25 hours at constant stirring (30-40 rpm). Afterwards the wetted powder is dried under reduced pressure (~500 mbar), jacket temperature 60°C and constant air flow until water content in the powder below 7 % is reached. The dried product (5.0 kg) was obtained in the form of powder after 35 hours of drying (LOD = 0.4%, KF = 6.4%, sesquihydrate polymorphic form).

Example 4: Preparation of pantoprazol sodium sesquihydrate

Wet pantoprazol sodium salt (8.9 kg, LOD 26.6%, KF 7.4%, obtained in Example 7), was charged into preheated filter dryer (Rosenmund, 0,1 $m^2$, 60°C) equipped with pneumatic-water spray nozzle, mechanical stirrer and open valves for venting acetone vapors. Wet material was pre-heated in filter dryer with jacket temperature 65°C and minimal stirring (cca 2-3 rpm) for 1.5 hours before water spraying. Water (1 L) was sprayed on powder with constant flow in 0.25 hours at constant stirring (30-40 rpm). Afterwards the wetted powder is dried under reduced pressure (~500 mbar), jacket temperature 60°C and constant air flow until water content in the powder below 7 % is reached. The dried product (5.2 kg) was obtained in the form of powder after 19 hours of drying (LOD = 0.4%, KF = 6.2%, sesquihydrate polymorphic form).

Example 5: Preparation of pantoprazol sodium sesquihydrate

Wet pantoprazol sodium salt (11.6 kg, LOD 31%, KF 6.7%, obtained in Example 8), was charged into preheated filter dryer (Rosenmund, 0,1 $m^2$, 60°C) equipped with mechanical stirrer, water spray nozzle with constant flow 85 ml/min and open valves for venting acetone vapors. Wet material was pre-heated in filter dryer with jacket temperature 60°C and minimal stirring (cca 2-3 rpm) and nitrogen purging for 1 hour before water spraying. Pre-heated water (1.3 L, 55 °C) was sprayed with constant flow on powder in three 4 minutes intervals with 5 minutes pauses in-between, at constant stirring speed (30-40 rpm). Afterwards the wetted powder was dried under reduced pressure (~500 mbar), jacket temperature 60°C and constant air flow until water content in the powder below 7 % is reached. The dried product (7.3 kg) was obtained in the form of powder after 30 hours of drying (KF = 6.2%, sesquihydrate polymorphic form).

Example 5a: Preparation of pantoprazol sodium sesquihydrate

Process was carried in the same way as in Example 5 with the exception that the wetted powder was dried under nitrogen flow.

Example 6: Preparation of pantoprazol sodium sesquihydrate

Wet pantoprazol sodium salt (11.6 kg, LOD 31%, KF 6.7%, obtained in Example 8), was charged into preheated filter dryer (Rosenmund, 0,1 $m^2$, 60°C) equipped with mechanical stirrer, water spray nozzle with constant flow 85 ml/min and open valves for venting acetone vapors. Wet material was pre-heated in filter dryer with jacket temperature 50°C and minimal stirring (cca 2-3 rpm) and nitrogen purging for 2 hour before water spraying. Pre-heated water (0,7 L, 55 °C) was sprayed with constant flow on powder in three 4 minutes intervals with 5 minutes pauses in-between, at constant stirring speed (30-40 rpm). Afterwards the wetted powder was dried under reduced pressure (~500 mbar), jacket temperature 60°C and constant air flow until water content in the powder below 7 % is reached. The dried product (7.2 kg) was obtained in the form of powder after 30 hours of drying (KF = 6.3%, sesquihydrate polymorphic form).)

Example 6a: Preparation of pantoprazol sodium sesquihydrate

Process was carried in the same way as in Example 6 with the exception that the wetted powder was dried under nitrogen flow.

Example 7: Procedure for preparation of wet pantoprazole sodium salt (pantoprazole sodium acetone-water solvates)

Wet pantoprazole sodium salt was obtained by the following preparation procedure, wherein the variable "M1" is 1 (M1 = 1):

Charge into the reactor acetone (4.2*M1 L), deionized water (0.3*M1 L) and NaOH (106.8*M1 g). Heat the solution to the reactor temperature 40-45 °C using jacket

temperature not more than 60 °C. During the heating, charge the dry pantoprazole, to the NaOH solution, prepared above (M1 kg).

Add active carbon (0.05*M1 kg) to the prepared solution of pantoprazole sodium and heat to reflux (55±5 °C) using jacket temperature not more then 75 °C and maintain at the reflux conditions for 30-40 min.

Filtrate the obtained solution through bag and pipe candle filter (0.2 μm) and wash the filter with hot acetone (0.88*M1 L). During the mixing or heating distill the amount of acetone added for the washing of filters.

Cool the obtained solution to the reactor temperature 0-5 °C with cooling rate of reactor temperature 0.1 - 0.8 °C/min. After the final crystallization temperature is reached (0-5 °C) maintain at this temperature for the time needed for the product to fully crystallize.

Isolate the product using centrifuge or pressure filter and wash the wet product with cooled acetone (0-5 °C, 0.88*M1 L). After the isolation is completed, pack and weigh the obtained wet product.

Example 8: Procedure for preparation of wet pantoprazole sodium salt (acetone-water solvates)

Wet pantoprazole sodium salt was obtained by the following preparation procedure, wherein the variable "M1" is 1 (M1 = 1):
Charge into the reactor acetone (4.2*M1 L), deionized water (0.3*M1 L) and NaOH (106.8*M1 g). Heat the solution to the reactor temperature 40-45 °C using jacket temperature not more than 60 °C. During the heating, charge the dry pantoprazole, to the NaOH solution, prepared above (M1 kg).

Add active carbon (0.05*M1 kg) to the prepared solution of pantoprazole sodium and heat to reflux (55±5 °C) using jacket temperature not more than 75 °C and maintain at the reflux conditions for 30-40 min.

Filtrate the obtained solution through bag and pipe candle filter (0.2 μm) and wash the filter with hot acetone (0.88*M1 L). During the mixing or heating distill the amount of acetone added for the washing of filters.

Cool the obtained solution to the reactor temperature 0-5 °C with cooling rate of reactor temperature 0.1 - 0.8 °C/min. During the cooling process, when the reactor temperature is between 40-42 °C, add the seeds of pure pantoprazole (sodium) monohydrate obtained in Example 9 (0.01*M1 kg). After the final crystallization temperature is reached (0-5 °C) maintain at this temperature for the next 2h. Isolate the product using centrifuge or pressure filter and wash the wet product with cooled acetone (0-5 °C, 0.88*M1, L). After the isolation is completed, pack and weigh the obtained wet product.

Example 9: Procedure for preparation of pantoprazole sodium salt monohydrate)

Pantoprazole sodium monohydrate salt was obtained by the following preparation procedure, wherein the variable "M1" is 1 (M1 = 1):

Charge into the reactor acetone (4.2*M1 L), deionized water (0.3*M1 L) and NaOH (106.8*M1 g). Heat the solution to the reactor temperature 40-45 °C using jacket temperature not more than 60 °C. During the heating, charge the dry pantoprazole, to the NaOH solution, prepared above (M1 kg).

Add active carbon (0.05*M1 kg) to the prepared solution of pantoprazole sodium and heat to reflux (55±5 °C) using jacket temperature not more then 75 °C and maintain at the reflux conditions for 30-40 min.

Filtrate the obtained solution through bag and pipe candle filter (0.2 μm) and wash the filter with hot acetone (0.88*M1 L). During the mixing or heating distill the amount of acetone added for the washing of filters.

Cool the obtained solution to the reactor temperature 0-5 °C with cooling rate of reactor temperature 0.1 - 0.8 °C/min. During the cooling process, when the reactor temperature is between 40-42 °C, add the seeds of pure pantoprazole (sodium) monohydrate (0.01*M1 kg). After the final crystallization temperature is reached (0-5 °C) maintain at this temperature for the time needed for the product to fully crystallize.

Isolate the product using centrifuge or pressure filter and wash the wet product with cooled acetone (0-5 °C, 0.88*M1 L). After the isolation is completed, pack and weigh the obtained wet product. Charge the wet cake into vacuum dryer. Start with drying at temperature 20-25°C and gradually raise it up to temperature 45°C. Use heating media up to 50°C. Circulate occasionally. For drying wet product you can use paddle dryer.

Example 10: Procedure for preparation of pantoprazole sodium salt monohydrate)

Pantoprazole sodium monohydrate salt was obtained by the following preparation procedure, wherein the variable "M1" is 1 (M1 = 1):
Charge into the reactor acetone (4.2*M1 L), deionized water (0.3*M1 L) and NaOH (106.8*M1 g). Heat the solution to the reactor temperature 40-45 °C using jacket temperature not more than 60 °C. During the heating, charge the dry pantoprazole, to the NaOH solution, prepared above (M1 kg).

Add active carbon (0.05*M1 kg) to the prepared solution of pantoprazole sodium and heat to reflux (55±5 °C) using jacket temperature not more then 75 °C and maintain at the reflux conditions for 30-40 min.

Filtrate the obtained solution through bag and pipe candle filter (0.2 μm) and wash the filter with hot acetone (0.88*M1 L). During the mixing or heating distill the amount of acetone added for the washing of filters.

Cool the obtained solution to the reactor temperature 0-5 °C with cooling rate of reactor temperature 0.1 - 0.8 °C/min. During the cooling process, when the reactor temperature is between 40-42 °C, add the seeds of pure pantoprazole (sodium) monohydrate obtained in Example 7 or the seeds of pure pantoprazole sodium

monohydrate (0.01*M1 kg). (Pantoprazole sodium monohydrate seeding crystals can for example also be obtained by one to the processes for preparing pantoprazole sodium monohydrate disclosed in EP 0 533 790 B1 or in another publication known in the art.) After the final crystallization temperature is reached (0-5 °C) maintain at this temperature for the next 2h.

Isolate the product using centrifuge or pressure filter and wash the wet product with cooled acetone (0-5 °C, 0.88*M1 L). After the isolation is completed, pack and weigh the obtained wet product.

Example 11: Analytical data

In the preceding examples, the water content was determined according to Karl-Fischer method according to Ph. Eur. 2.5.12., e.g. as indicated in European Pharmacopoeia 7.0, (or according to USP 921). The indication KF being an abbreviation for the water content determined according to Karl-Fischer method; the percentages indicated being weight percentages.

Furthermore, in the preceding examples a Loss on Drying (LOD) analysis was carried out with a temperature of 120°C as setpoint; the percentages indicated being weight percentages. This Loss on Drying analysis may be used for determining the amount of compounds of high and intermediate volatility in the sample, in particular for determining the amount of organic solvents (especially without bound water) in the sample.

If not explicitly indicated otherwise, X-ray powder diffraction patterns were/can be obtained by PANalytical PW3040/60 X'Pert PRO diffractometer using CuK$\alpha$_ radiation of 1,541874 Å.

## Claims

**1.** Process for the preparation of pantoprazole sodium sesquihydrate, said process comprising:

a) heating pantoprazole sodium to a temperature in the range from 40°C to 60°C, the pantoprazole sodium being at least partially present in solid form;
b) adding water to the pantoprazole sodium of step a) under agitation to obtain a mixture comprising solid pantoprazole sodium and water;
c) drying the mixture comprising solid pantoprazole sodium and water under agitation; and
d) recovering pantoprazole sodium sesquihydrate.

**2.** Process for the preparation of pantoprazole sodium sesquihydrate according to claim 1, **characterized in that** the amount of water added in step b) is at least 1 wt.-%, preferably at least 5 wt.-%, more preferably at least 10 wt.-%, each based on the total weight of pantoprazole sodium.

**3.** Process for the preparation of pantoprazole sodium sesquihydrate according to any one of the preceding claims, **characterized in that** the water addition time period in step b) is a time period of at least 1 minute, preferably at least 4 minutes, more preferred at least 10 minutes.

**4.** Process for the preparation of pantoprazole sodium sesquihydrate according to any one of the preceding claims, **characterized in that** the water addition according to step b) is or comprises spraying the water onto the pantoprazole sodium.

**5.** Process for the preparation of pantoprazole sodium sesquihydrate according to any one of the preceding claims, **characterized in that** the heating of pantoprazole sodium in step a) is carried out for a time period of at least 1 minute, preferably for a time period of at least about 3 minutes to about 24 hours, more preferably for a time period of about 0.4 hours to about 3 hours, especially for a time period of about 0.5 hours to about 2 hours.

**6.** Process for the preparation of pantoprazole sodium sesquihydrate according to any one of the preceding claims, **characterized in that** the pantoprazole sodium subjected to heating in step a) is or comprises one or more pantoprazole sodium solvates, in particular is or comprises at least one of one or more pantoprazole sodium hydrates, one or more pantoprazole sodium solvates containing at least one organic solvent and optionally water, and mixtures thereof.

**7.** Process for the preparation of pantoprazole sodium sesquihydrate according to any one of the preceding claims, **characterized in that** the pantoprazole sodium to be subjected to heating in step a) is present in a mixture comprising at least one solvent and pantoprazole sodium, preferably in a mixture comprising at least one solvent and solid pantoprazole sodium, more preferably in a mixture comprising at least one solvent and one or more pantoprazole sodium solvates, in particular in a mixture comprising at least one solvent and at least one of one or more pantoprazole sodium hydrates, one or more pantoprazole sodium solvates containing at least one organic solvent and optionally water, and mixtures thereof,
and wherein preferably
said at least one solvent is at least one or both of water and acetone, and optionally one or more further solvents.

**8.** Process for the preparation of pantoprazole sodium sesquihydrate according to any one of the preceding claims, **characterized in that** during the heating of pantoprazole sodium in step a), the pantoprazole sodium is subjected to agitation, and/or

**characterized in that** the adding of water to the pantoprazole sodium is carried out continuously or intermittently, and/or
**characterized in that** the temperature in step b) and/or in step c) is adjusted to a temperature of at least 30°C, preferably at least 35°C, more preferably at least 40°C, and/or
**characterized in that** the drying of the mixture comprising solid pantoprazole sodium and water (step c)) is carried out until the mixture comprising solid pantoprazole sodium and water has a water content of below 7,5 wt.-%, preferably below 7 wt.-%, each based on the weight of pantoprazole sodium present in the mixture.

**9.** Process for the preparation of pantoprazole sodium sesquihydrate according to any one of the preceding claims, **characterized in that** steps a) to c) are carried out in situ, preferably in situ in a filter dryer or a high-shear mixer, and/or
**characterized in that** the drying in step c) is carried out over a time period of at least 2 hours, optionally 6 hours to 10 days, and/or
**characterized in that** the pantoprazole sodium to be subjected to heating in step a) is present in a mixture comprising pantoprazole sodium, acetone, and optionally water, said mixture containing less than 90 wt.-% of acetone, preferably less than 50 wt.-% of acetone, more preferably less than 40 wt.-%, especially between less than 40 wt.-% and at least 0,1 wt.-% of acetone, each based on the weight of the mixture.

**10.** Process for the preparation of pantoprazole sodium sesquihydrate according to any one of the preceding claims, **characterized in that** the pantoprazole sodium to be subjected to heating in step a) is pantoprazole sodium free of pantoprazole sodium sesquihydrate.

**11.** Process for the preparation of pantoprazole sodium sesquihydrate according to any one of the preceding claims 1 to 9, **characterized in that** the pantoprazole sodium to be subjected to heating in step a) is pantoprazole sodium comprising less than 98 mole %, in particular less than 90 mole %, especially less than 60 mole %, further especially less than 20 mole %, still further especially less than 10 mole % of pantoprazole sodium sesquihydrate, based on the total number of moles of pantoprazole sodium.

**12.** Process for the preparation of pantoprazole sodium sesquihydrate according to any one of the preceding claims 1 to 9, and 11, **characterized in that** the pantoprazole sodium to be subjected to heating in step a) is a mixture comprising one or more pantoprazole sodium solvates, the amount of pantoprazole sodium sesquihydrate in said mixture being less than 98 mole %, in particular less than 90 mole %, especially less than 60 mole %, further especially less than 20 mole %, still further especially less than 10 mole %, based on the total number of moles of pantoprazole sodium in said mixture.

**13.** Process for the preparation of pantoprazole sodium sesquihydrate according to any one of the preceding claims, **characterized in that** the pantoprazole sodium to be subjected to heating in step a) comprises or consists of pantoprazole sodium present in a form different from pantoprazole sodium sesquihydrate.

**14.** Process for the preparation of pantoprazole sodium sesquihydrate according to claim 13, **characterized in that** the pantoprazole sodium to be subjected to heating in step a) comprises or consists of pantoprazole sodium present in a form different from pantoprazole sodium sesquihydrate, and
**in that** carrying out steps a) to c) converts pantoprazole sodium present in a form different from pantoprazole sodium sesquihydrate into pantoprazole sodium sesquihydrate.

**Patentansprüche**

**1.** Verfahren zur Herstellung von Pantoprazol-Natrium-Sesquihydrat, wobei das Verfahren umfasst:

a) Erwärmen von Pantoprazol-Natrium auf eine Temperatur im Bereich von 40°C bis 60°C, wobei das Pantoprazol-Natrium mindestens teilweise in fester Form vorliegt;
b) Geben von Wasser zu dem Pantoprazol-Natrium von Schritt a) unter Bewegung, wobei ein Gemisch, umfassend festes Pantoprazol-Natrium und Wasser, erhalten wird;
c) Trocknen des Gemisches, umfassend festes Pantoprazol-Natrium und Wasser, unter Bewegung; und
d) Gewinnen von Pantoprazol-Natrium-Sesquihydrat.

**2.** Verfahren zur Herstellung von Pantoprazol-Natrium-Sesquihydrat gemäß Anspruch 1, **dadurch gekennzeichnet, dass** die in Schritt b) zugegebene Menge an Wasser mindestens 1 Gew.-%, bevorzugt mindestens 5 Gew.-%, stärker bevorzugt mindestens 10 Gew.-%, jeweils bezogen auf das Gesamtgewicht von Pantoprazol-Natrium, beträgt.

**3.** Verfahren zur Herstellung von Pantoprazol-Natrium-Sesquihydrat gemäß einem der vorhergehenden Ansprüche, **dadurch gekennzeichnet, dass** die Wasserzugabe-Zeitdauer in Schritt b) eine Zeitdauer von mindestens 1 Minute, bevorzugt mindestens 4 Minuten, stärker bevorzugt mindestens 10 Minuten

ist.

4. Verfahren zur Herstellung von Pantoprazol-Natrium-Sesquihydrat gemäß einem der vorhergehenden Ansprüche, **dadurch gekennzeichnet, dass** die Wasserzugabe gemäß Schritt b) Sprühen des Wassers auf das Pantoprazol-Natrium ist oder umfasst.

5. Verfahren zur Herstellung von Pantoprazol-Natrium-Sesquihydrat gemäß einem der vorhergehenden Ansprüche, **dadurch gekennzeichnet, dass** das Erwärmen von Pantoprazol-Natrium in Schritt a) für eine Zeitdauer von mindestens 1 Minute, bevorzugt für eine Zeitdauer von mindestens etwa 3 Minuten bis etwa 24 Stunden, stärker bevorzugt für eine Zeitdauer von etwa 0,4 Stunden bis etwa 3 Stunden, insbesondere für eine Zeitdauer von etwa 0,5 Stunden bis etwa 2 Stunden durchgeführt wird.

6. Verfahren zur Herstellung von Pantoprazol-Natrium-Sesquihydrat gemäß einem der vorhergehenden Ansprüche, **dadurch gekennzeichnet, dass** das Pantoprazol-Natrium, welches in Schritt a) Erwärmen ausgesetzt wird, ein oder mehr Pantoprazol-Natrium-Solvate ist oder umfasst, insbesondere mindestens eines von einem oder mehr Pantoprazol-Natrium-Hydraten, einem oder mehr Pantoprazol-Natrium-Solvaten, enthaltend mindestens ein organisches Lösungsmittel und gegebenenfalls Wasser, und Gemischen davon ist oder umfasst.

7. Verfahren zur Herstellung von Pantoprazol-Natrium-Sesquihydrat gemäß einem der vorhergehenden Ansprüche, **dadurch gekennzeichnet, dass** das Pantoprazol-Natrium, welches in Schritt a) Erwärmen ausgesetzt wird, in einem Gemisch, umfassend mindestens ein Lösungsmittel und Pantoprazol-Natrium, bevorzugt in einem Gemisch, umfassend mindestens ein Lösungsmittel und festes Pantoprazol-Natrium, stärker bevorzugt in einem Gemisch, umfassend mindestens ein Lösungsmittel und ein oder mehr Pantoprazol-Natrium-Solvate, insbesondere in einem Gemisch, umfassend mindestens ein Lösungsmittel und mindestens eines von einem oder mehr Pantoprazol-Natrium-Hydraten, einem oder mehr Pantoprazol-Natrium-Solvaten, enthaltend mindestens ein organisches Lösungsmittel und gegebenenfalls Wasser, und Gemischen davon, vorliegt,
und wobei bevorzugt
das mindestens eine Lösungsmittel mindestens eines oder beide von Wasser und Aceton, und gegebenenfalls ein oder mehr weitere Lösungsmittel ist.

8. Verfahren zur Herstellung von Pantoprazol-Natrium-Sesquihydrat gemäß einem der vorhergehenden Ansprüche, **dadurch gekennzeichnet, dass** während dem Erwärmen von Pantoprazol-Natrium in Schritt a) das Pantoprazol-Natrium Bewegung ausgesetzt wird, und/oder **dadurch gekennzeichnet, dass** das Geben von Wasser zu dem Pantoprazol-Natrium kontinuierlich oder intermittierend durchgeführt wird, und/oder **dadurch gekennzeichnet, dass** die Temperatur in Schritt b) und/oder in Schritt c) auf eine Temperatur von mindestens 30°C, bevorzugt mindestens 35°C, stärker bevorzugt mindestens 40°C eingestellt wird, und/oder **dadurch gekennzeichnet, dass** das Trocknen des Gemisches, umfassend festes Pantoprazol-Natrium und Wasser, (Schritt c) durchgeführt wird, bis das Gemisch, umfassend festes Pantoprazol-Natrium und Wasser, einen Wassergehalt von unter 7,5 Gew.-%, bevorzugt unter 7 Gew.-%, jeweils bezogen auf das Gewicht von Pantoprazol-Natrium, welches in dem Gemisch vorliegt, aufweist.

9. Verfahren zur Herstellung von Pantoprazol-Natrium-Sesquihydrat gemäß einem der vorhergehenden Ansprüche, **dadurch gekennzeichnet, dass** Schritte a) bis c) in situ, bevorzugt in situ in einem Filtertrockner oder einer Mischvorrichtung mit hoher Scherung durchgeführt werden, und/oder **dadurch gekennzeichnet, dass** das Trocknen in Schritt c) über eine Zeitdauer von mindestens 2 Stunden, gegebenenfalls 6 Stunden bis 10 Tage durchgeführt wird, und/oder
**dadurch gekennzeichnet, dass** das Pantoprazol-Natrium, welches in Schritt a) Erwärmen ausgesetzt wird, in einem Gemisch, umfassend Pantoprazol-Natrium, Aceton und gegebenenfalls Wasser, vorliegt, wobei das Gemisch weniger als 90 Gew.-% Aceton, bevorzugt weniger als 50 Gew.-% Aceton, stärker bevorzugt weniger als 40 Gew.-%, insbesondere zwischen weniger als 40 Gew.-% und mindestens 0,1 Gew.-% Aceton, jeweils bezogen auf das Gewicht des Gemisches, enthält.

10. Verfahren zur Herstellung von Pantoprazol-Natrium-Sesquihydrat gemäß einem der vorhergehenden Ansprüche, **dadurch gekennzeichnet, dass** das Pantoprazol-Natrium, welches in Schritt a) Erwärmen ausgesetzt wird, Pantoprazol-Natrium, das frei von Pantoprazol-Natrium-Sesquihydrat ist, ist.

11. Verfahren zur Herstellung von Pantoprazol-Natrium-Sesquihydrat gemäß einem der vorhergehenden Ansprüche 1 bis 9, **dadurch gekennzeichnet, dass** das Pantoprazol-Natrium, welches in Schritt a) Erwärmen ausgesetzt wird, Pantoprazol-Natrium, umfassend weniger als 98 Mol-%, insbesondere weniger als 90 Mol-%, insbesondere weniger als 60 Mol-%, ferner insbesondere weniger als 20 Mol-%, noch weiter besonders weniger als 10 Mol-% Pantoprazol-Natrium-Sesquihydrat, bezogen auf die Gesamtmolzahl von Pantoprazol-Natrium, ist.

12. Verfahren zur Herstellung von Pantoprazol-Natrium-Sesquihydrat gemäß einem der vorhergehenden Ansprüche 1 bis 9 und 11, **dadurch gekennzeichnet, dass** das Pantoprazol-Natrium, welches in Schritt a) Erwärmen ausgesetzt wird, ein Gemisch, umfassend ein oder mehr Pantoprazol-Natrium-Solvate, ist, wobei die Menge an Pantoprazol-Natrium-Sesquihydrat in dem Gemisch weniger als 98 Mol-%, insbesondere weniger als 90 Mol-%, insbesondere weniger als 60 Mol-%, ferner insbesondere weniger als 20 Mol-%, noch weiter besonders weniger als 10 Mol-%, bezogen auf die Gesamtmolzahl von Pantoprazol-Natrium in dem Gemisch, beträgt.

13. Verfahren zur Herstellung von Pantoprazol-Natrium-Sesquihydrat gemäß einem der vorhergehenden Ansprüche, **dadurch gekennzeichnet, dass** das Pantoprazol-Natrium, welches in Schritt a) Erwärmen ausgesetzt wird, umfasst oder besteht aus Pantoprazol-Natrium, das in einer Form, welche unterschiedlich zu Pantoprazol-Natrium-Sesquihydrat ist, vorliegt.

14. Verfahren zur Herstellung von Pantoprazol-Natrium-Sesquihydrat gemäß Anspruch 13, **dadurch gekennzeichnet, dass** das Pantoprazol-Natrium, welches in Schritt a) Erwärmen ausgesetzt wird, umfasst oder besteht aus Pantoprazol-Natrium, das in einer Form, welche unterschiedlich zu Pantoprazol-Natrium-Sesquihydrat ist, vorliegt, und
dass das Durchführen von Schritten a) bis c) Pantoprazol-Natrium, das in einer Form, welche unterschiedlich zu Pantoprazol-Natrium-Sesquihydrat ist, vorliegt, in Pantoprazol-Natrium-Sesquihydrat umwandelt.

## Revendications

1. Procédé de préparation de pantoprazole sodique sesquihydraté, ledit procédé comprenant le fait :

   a) de chauffer du pantoprazole sodique à une température se trouvant dans la plage allant de 40°C à 60°C, le pantoprazole sodique étant au moins partiellement présent sous forme solide ;
   b) d'ajouter de l'eau au pantoprazole sodique de l'étape a) sous agitation pour obtenir un mélange comprenant du pantoprazole sodique solide et de l'eau ;
   c) de sécher le mélange comprenant du pantoprazole sodique solide et de l'eau sous agitation ; et
   d) de récupérer le pantoprazole sodique sesquihydraté.

2. Procédé de préparation de pantoprazole sodique sesquihydraté selon la revendication 1, **caractérisé en ce que** la quantité d'eau ajoutée dans l'étape b) est d'au moins 1% en poids, de préférence d'au moins 5% en poids, plus préférablement d'au moins 10% en poids, chacun par rapport au poids total de pantoprazole sodique.

3. Procédé de préparation de pantoprazole sodique sesquihydraté selon l'une quelconque des revendications précédentes, **caractérisé en ce que** la durée d'addition d'eau dans l'étape b) est une durée d'au moins 1 minute, de préférence d'au moins 4 minutes, plus préférablement d'au moins 10 minutes.

4. Procédé de préparation de pantoprazole sodique sesquihydraté selon l'une quelconque des revendications précédentes, **caractérisé en ce que** l'addition d'eau selon l'étape b) est ou comprend la pulvérisation de l'eau sur le pantoprazole sodique.

5. Procédé de préparation de pantoprazole sodique sesquihydraté selon l'une quelconque des revendications précédentes, **caractérisé en ce que** le chauffage du pantoprazole sodique dans l'étape a) est réalisé pendant une durée d'au moins 1 minute, de préférence pendant une durée d'au moins environ 3 minutes à environ 24 heures, plus préférablement pendant une durée d'environ 0,4 heure à environ 3 heures, en particulier pendant une durée d'environ 0,5 heure à environ 2 heures.

6. Procédé de préparation de pantoprazole sodique sesquihydraté selon l'une quelconque des revendications précédentes, **caractérisé en ce que** le pantoprazole sodique soumis au chauffage dans l'étape a) est ou comprend un ou plusieurs solvate(s) de pantoprazole sodique, notamment est ou comprend au moins un d'un ou de plusieurs hydrate(s) de pantoprazole sodique, un ou plusieurs solvate(s) de pantoprazole sodique contenant au moins un solvant organique et éventuellement de l'eau, et leurs mélanges.

7. Procédé de préparation de pantoprazole sodique sesquihydraté selon l'une quelconque des revendications précédentes, **caractérisé en ce que** le pantoprazole sodique devant être soumis au chauffage dans l'étape a) est présent dans un mélange comprenant au moins un solvant et du pantoprazole sodique, de préférence dans un mélange comprenant au moins un solvant et du pantoprazole sodique solide, plus préférablement dans un mélange comprenant au moins un solvant et un ou plusieurs solvate(s) de pantoprazole sodique, notamment dans un mélange comprenant au moins un solvant et au moins un parmi un ou plusieurs hydrate(s) de pantoprazole sodique, un ou plusieurs solvate(s) de pantoprazole sodique contenant au moins un solvant

organique et éventuellement de l'eau, et leurs mélanges,

et dans lequel de préférence

ledit au moins un solvant est au moins l'un(e) parmi l'eau et l'acétone ou les deux, et éventuellement un ou plusieurs solvant(s) supplémentaire(s).

**8.** Procédé de préparation de pantoprazole sodique sesquihydraté selon l'une quelconque des revendications précédentes, **caractérisé en ce que** lors du chauffage du pantoprazole sodique dans l'étape a), le pantoprazole sodique est soumis à une agitation, et/ou

**caractérisé en ce que** l'addition d'eau au pantoprazole sodique est réalisée de manière continue ou intermittente, et/ou

**caractérisé en ce que** la température dans l'étape b) et/ou dans l'étape c) est ajustée à une température d'au moins 30°C, de préférence d'au moins 35°C, plus préférablement d'au moins 40°C, et/ou

**caractérisé en ce que** le séchage du mélange comprenant du pantoprazole sodique solide et de l'eau (étape c) est réalisé jusqu'à ce que le mélange comprenant du pantoprazole sodique solide et de l'eau ait une teneur en eau inférieure à 7,5% en poids, de préférence inférieure à 7% en poids, chacune par rapport au poids de pantoprazole sodique présent dans le mélange.

**9.** Procédé de préparation de pantoprazole sodique sesquihydraté selon l'une quelconque des revendications précédentes, **caractérisé en ce que** les étapes a) à c) sont réalisées in situ, de préférence in situ dans un filtre-sécheur ou un mélangeur à cisaillement élevé, et/ou

**caractérisé en ce que** le séchage dans l'étape c) est réalisé sur une durée d'au moins 2 heures, éventuellement de 6 heures à 10 jours, et/ou

**caractérisé en ce que** le pantoprazole sodique devant être soumis à un chauffage dans l'étape a) est présent dans un mélange comprenant du pantoprazole sodique, de l'acétone et éventuellement de l'eau, ledit mélange contenant moins de 90% en poids d'acétone, de préférence moins de 50% en poids % d'acétone, plus préférablement moins de 40% en poids, en particulier entre moins de 40% en poids et au moins 0,1% en poids d'acétone, chacun par rapport au poids du mélange.

**10.** Procédé de préparation de pantoprazole sodique sesquihydraté selon l'une quelconque des revendications précédentes, **caractérisé en ce que** le pantoprazole sodique devant être soumis à un chauffage dans l'étape a) est un pantoprazole sodique exempt de pantoprazole sodique sesquihydraté.

**11.** Procédé de préparation de pantoprazole sodique sesquihydraté selon l'une quelconque des revendications 1 à 9 précédentes, **caractérisé en ce que** le pantoprazole sodique devant être soumis à un chauffage dans l'étape a) est un pantoprazole sodique comprenant moins de 98% en mole, notamment moins de 90% en mole, en particulier moins de 60% en mole, plus particulièrement moins de 20% en mole, encore plus particulièrement moins de 10% en mole de pantoprazole sodique sesquihydraté, par rapport au nombre total de moles de pantoprazole sodique.

**12.** Procédé de préparation de pantoprazole sodique sesquihydraté selon l'une quelconque des revendications précédentes 1 à 9, et 11, **caractérisé en ce que** le pantoprazole sodique devant être soumis à un chauffage dans l'étape a) est un mélange comprenant un ou plusieurs solvate(s) de pantoprazole sodique, la quantité de pantoprazole sodique sesquihydraté dans ledit mélange étant inférieure à 98% en mole, notamment inférieure à 90% en mole, en particulier inférieure à 60% en mole, plus particulièrement inférieure à 20% en mole, encore plus particulièrement inférieure à 10% en mole, par rapport au nombre total de moles de pantoprazole sodique dans ledit mélange.

**13.** Procédé de préparation de pantoprazole sodique sesquihydraté selon l'une quelconque des revendications précédentes, **caractérisé en ce que** le pantoprazole sodique devant être soumis à un chauffage dans l'étape a) comprend ou consiste en du pantoprazole sodique présent sous une forme différente du pantoprazole sodique sesquihydraté.

**14.** Procédé de préparation de pantoprazole sodique sesquihydraté selon la revendication 13, **caractérisé en ce que** le pantoprazole sodique devant être soumis à un chauffage dans l'étape a) comprend ou consiste en du pantoprazole sodique présent sous une forme différente du pantoprazole sodique sesquihydraté, et

**en ce que** la réalisation des étapes a) à c) transforme le pantoprazole sodique présent sous une forme différente du pantoprazole sodique sesquihydraté en pantoprazole sodique sesquihydraté.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004080961 A
- WO 2004111029 A
- US 20040186139 A
- WO 2004056804 A
- WO 2004063188 A
- EP 1300406 A
- WO 2007017244 A
- WO 2006040778 A
- WO 2007068925 A
- WO 2007026188 A
- WO 03097606 A
- WO 2004100949 A2
- EP 2030973 A1
- CN 103467451 A
- US 2004186139 A1
- WO 2009075516 A2
- US 6410569 B1
- EP 0533790 B1
- WO 2004056804 A2
- US 20040186139 A1


### Non-patent literature cited in the description


- *J. Med. Chemistry,* 1992, vol. 35, 1049-1057
- *Anal. Profiles of Drug Substances - Pantoprazole Sodium,* vol. 29, 213-259
- *Chin. Pharm,* August 1999, vol. 34 (8), 564-565
- Formation Mechanism and Phase Transformation Behaviors of Pantoprazole Sodium Heterosolvate. *ORGANIC PROCESS RESEARCH AND DEVELOPMENT,* 2015, vol. 19, 1752-1759
- Development of Suitable Plant-Scale Drying Conditions That Prevent API Agglomeration and Dehydration. *ORGANIC PROCESS RESEARCH AND DEVELOPMENT,* 2016, vol. 20, 51-58
- Glass-lined Filter/Dryers. Rosenmund VTA AG, 05 February 2016
- *International Journal of Pharmaceutics,* 2005, vol. 291, 59-68
- **V. ZUPANČIČ ; N. OGRAJŠEK ; B. KOTAR-JORDAN ; F. VREČER.** Physical characterization of pantoprazole sodium hydrates. *International Journal of Pharmaceutics,* 2005, vol. 291, 59-68
- *Journal of Pharmaceutics,* 2005, vol. 291, 59-68
- US Pharmacopoeia's (USP) publication USP-NF. The United States Pharmacopoeial Convention, Inc, 2004
- European Pharmacopoeia 7.0